# EUROPEAN PATENT SPECIFICATION

(11) **EP 3 390 350 B1**
(45) Date of publication and mention of the grant of the patent: **13.09.2023**
(21) Application number: 16819384.5
(22) Date of filing: 23.11.2016
(51) Int. Cl.: C07C 253/34, C07C 255/08

(54) **RECOVERY COLUMN CONTROL**
RÜCKGEWINNUNGSSÄULENSTEUERUNG
RÉGULATION D'UNE COLONNE DE RÉCUPÉRATION

(30) Priority: 17.12.2015 CN 201510947424
(43) Date of publication of application: 24.10.2018
(73) Proprietor: Ineos Europe AG, 1180 Rolle (CH)
(72) Inventor: SHOUCHE, Manoj Shrikant, Pearland Texas 77584 (US); MCDONEL, Timothy Robert, Elburn Illinois 60119 (US); COUCH, Jay Robert, Naperville Illinois 60564 (US)
(74) Representative: King, Alex
(86) International application number: PCT/US2016/063538
(87) International publication number: WO 2017/105803

(56) References cited:
- DE-A1- 1 468 659
- US-A- 3 445 347

## Description

An extractive distillation process is provided for separating acrylonitrile from a mixture of acrylonitrile and acetonitrile. More specifically, the process includes contacting the mixture with aqueous solvent to provide an acrylonitrile-water azeotrope and separating the acrylonitrile-water azeotrope from the acetonitrile.

### BACKGROUND

Recovery and purification systems for acrylonitrile are known, see for example U.S. Pat. Nos. 4,234,510; 3,936,360; 3,885,928; 3,433,822; and 3,399,120. Typically, propylene, ammonia and air are reacted in a vapor phase with an ammoxidation catalyst. The vaporous reactor effluent is then passed to a quench system wherein the reactor effluent is directly contacted with an aqueous quenching liquid, usually water. This quenching removes unreacted ammonia and heavy polymers. The quenched gases then proceed to an absorption column.

In the absorber, the gases are directly contacted with an absorbing liquid, again usually water. The water, acrylonitrile, acetonitrile, HCN and associated impurities leave the bottom of the absorber in an aqueous solution. Inert gases are removed from the top of the absorber. The aqueous solution then proceeds to a recovery column. This column removes acetonitrile from the aqueous solution through extractive distillation.

The recovery column in the acrylonitrile manufacturing process plays a key role in recovering crude acrylonitrile from the rich water containing acrylonitrile, hydrogen cyanide (HCN), acetonitrile, water, and other unwanted impurities made during the ammoxidation process in the reactors. A process of extractive distillation is used in the recovery column to separate the acrylonitrile and acetonitrile, which have very close boiling points. Solvent water is added in the top of the column to facilitate the extractive distillation. Acetonitrile and other heavy impurities are forced down the column with the solvent water, whereas, HCN and the acrylonitrile-water azeotrope are recovered from the top of the column as crude acrylonitrile. The crude acrylonitrile is then processed further in the purification section to recover pure acrylonitrile. The bottom section of the column is used to recover crude acetonitrile by a stripping process using steam in the reboilers of the recovery column. US 3,445,347, for example describes an extractive distillation process for the separation of acrylonitrile and acetonitrile in which there is withdrawn an acrylonitrile-water azeotrope as an overhead fraction, a bottom fraction containing water and from about 0.2% to 8% by weight of acetonitrile, and an intermediate impurities fraction containing water together with higher nitriles and alcohols.

The operating performance of the column to recover crude acrylonitrile from the rich water depends on multiple process parameters such as, the amount of solvent water added, the temperature of the solvent water, the amount of steam used in the reboilers, and the temperature of the rich water. All these variables have a strong influence on the column overhead composition, and also the column loading. This multivariate nature of the relationship between the process variables poses a problem of properly controlling the key variables and optimizing them. Traditional control methods (such as PID control) induce significant variations in the variables and result in suboptimal column performance. Optimizing the column performance is very important to save steam usage, and to increase plant throughput. For example, using excess solvent water does reduce the amount of impurities such as oxazole and acetonitrile in the crude acrylonitrile recovered, but increases the load the column and hence, increases the steam usage. This reduces the capacity of the column to treat more rich water, which reduces the overall plant throughput. Less solvent water on the other hand, increases the amount of impurities and also acetonitrile in the crude acrylonitrile. Excessive amounts of these impurities end up in the final product, resulting in failure to meet acrylonitrile product specification requirement.

### SUMMARY

An extractive distillation process includes providing a mixture that includes acrylonitrile and acetonitrile to at least one distillation column; contacting the mixture that includes acrylonitrile and acetonitrile with aqueous solvent to provide an acrylonitrile-water azeotrope; and separating the acrylonitrile-water azeotrope from the acetonitrile to provide an overhead stream, a sidestream and a bottoms stream.

The overhead stream comprises the acrylonitrile-water azeotrope, 0.05 weight percent or less acetonitrile, and 70 to 90 weight percent acrylonitrile, the bottoms stream comprises 0 to 0.0075 weight percent acetonitrile, and the side stream comprises 5 to 70 weight percent acetonitrile and 1 weight percent or less acrylonitrile. The weight percent acetonitrile in the overhead stream and the weight percent acrylonitrile in the sidestream are controlled based on the flow of the aqueous solvent added to the distillation column and a temperature of the distillation column

An extractive distillation column control process includes providing a mixture that includes acrylonitrile and acetonitrile to at least one distillation column; contacting the mixture that includes acrylonitrile and acetonitrile with aqueous solvent to provide an acrylonitrile-water azeotrope; separating the acrylonitrile-water azeotrope from the acetonitrile to provide an overhead stream, a sidestream and a bottoms stream; and controlling an amount of acetonitrile in the overhead stream and the amount of acrylonitrile in the sidestream using an advanced process controller based on model predictive control to determine simultaneous control actions for manipulated variables in order to optimize at least one set of parameters while controlling at least one set of controlled variables. The overhead stream comprises the acrylonitrile-water azeotrope, 0.05 weight percent or less acetonitrile, and 70 to 90 weight percent acrylonitrile, the bottoms stream comprises 0 to 0.0075 weight percent acetonitrile, and the side stream comprises 5 to 70 weight percent acetonitrile and 1 weight percent or less acrylonitrile. In this aspect, the set of manipulated variables includes an amount of the aqueous solvent added to the distillation column and distillation column temperature and the set of controlled variable includes the amount of acetonitrile in the overhead and the amount of acrylonitrile in the sidestream. The controlling of the at least one set of controlled variables includes controlling the amount of aqueous solvent added to the distillation column and the distillation column temperature.

### BRIEF DESCRIPTION OF FIGURES

The above and other aspects, features and advantages of several aspects of the process will be more apparent from the following figures.
Figure 1 provides a general view of a recovery column;
Figure 2 illustrates another view of a recovery column;
Figure 3 illustrates a recovery column that includes an acetonitrile concentration zone; and
Figure 4 illustrates a recovery column temperature profile.

Corresponding reference characters indicate corresponding components throughout the several views of the drawings. Skilled artisans will appreciate that elements in the figures are illustrated for simplicity and clarity and have not necessarily been drawn to scale. For example, the dimensions of some of the elements in the figures may be exaggerated relative to other elements to help to improve understanding of various aspects. Also, common but well-understood elements that are useful or necessary in a commercially feasible aspect are often not depicted in order to facilitate a less obstructed view of these various aspects.

### DETAILED DESCRIPTION

The following description is not to be taken in a limiting sense, but is made merely for the purpose of describing the general principles of exemplary embodiments. The scope of the invention should be determined with reference to the claims.

### Recovery Column

Any type of recovery column may be utilized in connection with the present process. Several types of recovery column configurations are described herein as examples.

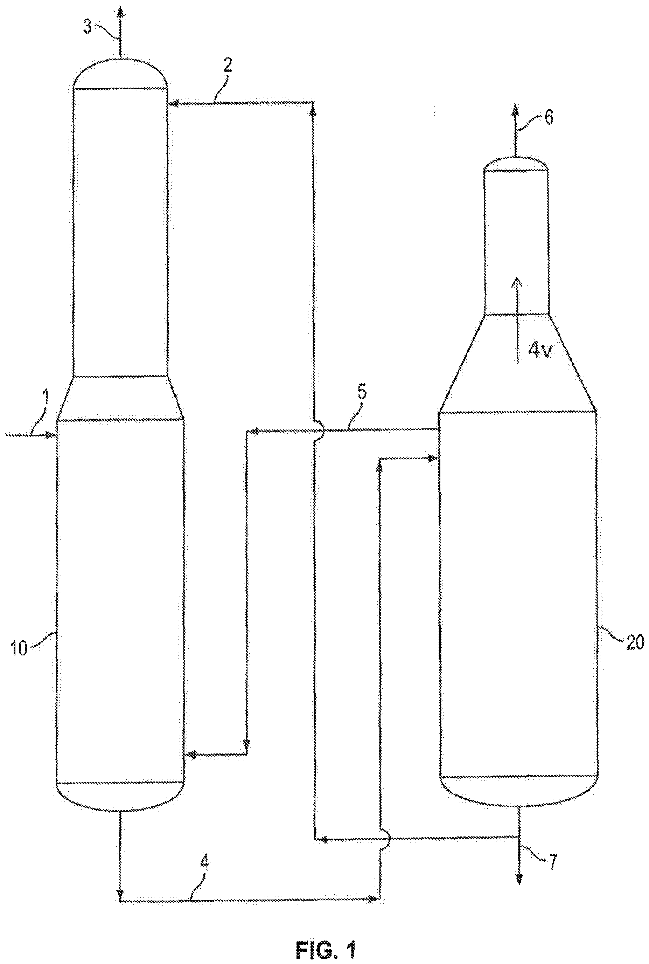

A conventional process for separation of acrylonitrile from acetonitrile is shown in FIG. 1. As shown in FIG. 1, feed stream 1 from an acrylonitrile absorber (not shown) is sent to first column 10. Feed stream 1 typically contains acrylonitrile, hydrogen cyanide (HCN), acetonitrile, and water. Water stream 2 that is substantially free of acetonitrile is recycled from at or near the bottom of second column 20 to an upper portion of first column 10 in order to facilitate the separation of acetonitrile from acrylonitrile and HCN by extractive distillation. Stream 3 containing acrylonitrile, HCN, and a portion of the water from feed 1 is removed from the top of first column 10. Liquid stream 4 containing water and acetonitrile is sent as feed from the bottom of first column 10 to second column 20. Vapor stream 5 from second column 20 is sent to first column 10 to provide the heat needed for distillation in first column 10. Vapor sidestream 4v moves up the second column 20 and includes acetonitrile. A crude acetonitrile stream 6 containing acetonitrile, water and small amounts of acrylonitrile and HCN is removed from the top of second column 20. The remaining water stream 7, which is substantially free of acrylonitrile, HCN, and acetonitrile and is not recycled as water stream 2 back to first column 10, is discharged from second column 20 at or near the bottom of second column 20.

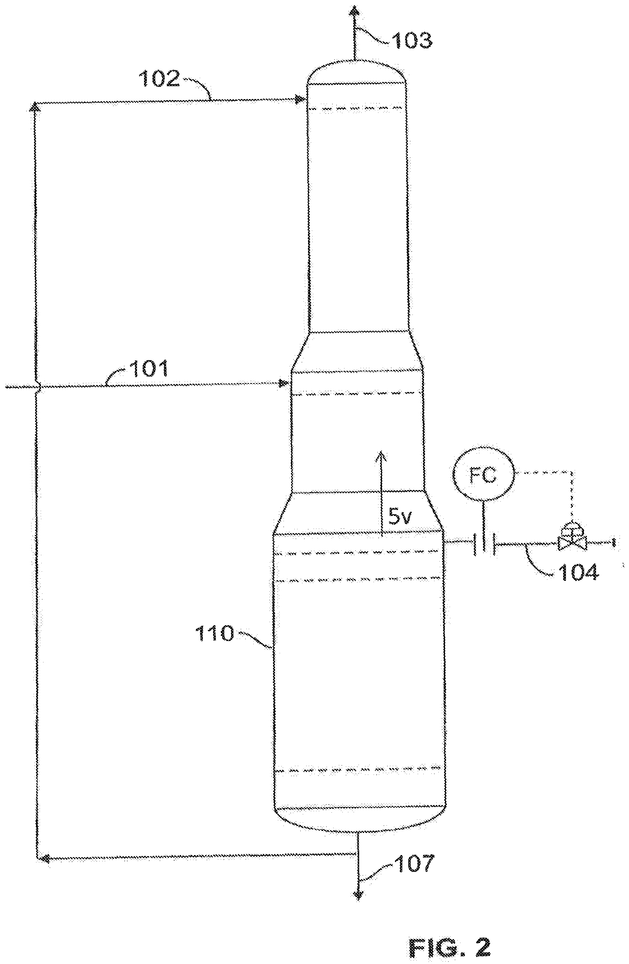

Another conventional process for separation of acrylonitrile from crude acetonitrile is shown in FIG. 2. As shown in FIG. 2, feed stream 101 from an acrylonitrile absorber (not shown) is sent to column 110. Feed stream 101 typically contains acrylonitrile, hydrogen cyanide (HCN), acetonitrile, and water. Bottoms stream 102 that is substantially free of acetonitrile is recycled from at or near the bottom of column 110 to the top of column 110 in order to facilitate the separation of acetonitrile from acrylonitrile and HCN by extractive distillation. The portion of bottoms stream 102 that is not recycled to the top of column 110 is discharged from column 110 as stream 107. Overhead stream 103 containing acrylonitrile, HCN, and a portion of the water from feed stream 101 is removed from the top of column 110. A vapor sidestream 5v moves up column 110 and a sidestream 104 (corresponding to 4v in FIG. 1) containing water and acetonitrile is removed from column 110.

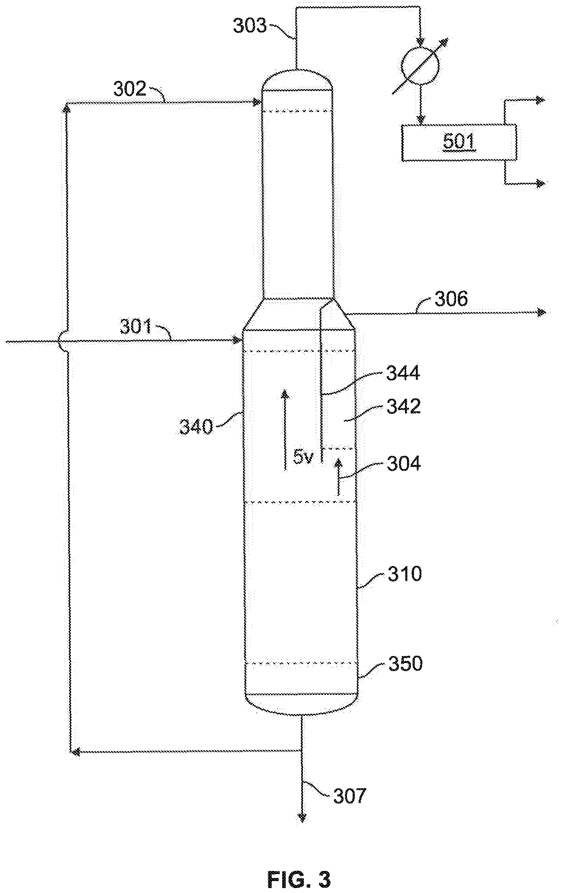

Another example of a recovery column is shown in FIG. 3. In this aspect, apparatus 300 includes column 310. Column 310 includes a top section 330, a middle section 340, and a bottom section 350. Middle section 340 of the single column 310 may be configured to receive feed stream 301. In one aspect, the column includes a top section and a middle section and a ratio of a diameter of the middle section to the top section is about 0.8 to about 1.2, in another aspect about 0.9 to about 1.1, in another aspect about 1.5 to about 2.5, in another aspect, about 1.75 to about 2.25, and in another aspect, about 1.8 to about 2. In one aspect, the column includes a middle section and a bottom section and a ratio of a diameter of the bottom section to the middle section is about 0.8 to about 1.2, in another aspect about 0.9 to about 1.1, in another aspect about 1.5 to about 2.5, in another aspect, about 1.75 to about 2.25, and in another aspect, about 1.8 to about 2. In one aspect, the column includes a top section and a bottom section and a ratio of a diameter of the bottom section to the top section is about 0.8 to about 1.2, in another aspect about 0.9 to about 1.1, in another aspect about 1.5 to about 2.5, in another aspect, about 1.75 to about 2.25, and in another aspect, about 1.8 to about 2. In one aspect, the top section, middle section and bottom section are each about 25 to about 40% of a height (tangent to tangent) of the recovery column.

As shown in FIG. 3, overhead stream 303 containing acrylonitrile, HCN and water is removed from the top of column 310. The overhead stream 303 is sent to a decanter 501. In this aspect, the process includes maintaining a decanter volume that is about 50 to about 70 percent of a daily average volume of the decanter. In another aspect, the process includes maintaining a decanter volume that is about 50 to about 60 percent, in another aspect, about 55 to about 70 percent, and in another aspect, about 55 to about 60 weight percent of a daily average volume of the decanter. In another aspect, an organic flow from the decanter is about 6 to about 11 percent of a volume of the acrylonitrile and acetonitrile mixture provided to the column. In another aspect, an organic flow form the decanter is about 6 to about 10 percent, in another aspect, about 8 to about 10 percent, and in another aspect, about 8.75 to about 10 percent.

As further shown in Fig. 3, the recovery column may include a crude acetonitrile concentration zone 342. The crude acetonitrile concentration zone 342 includes internal vertical baffle 344. The crude acetonitrile concentration zone may include a plurality of trays. The trays are located at different heights of the column, and each tray includes a horizontal plane extending across a cross section of crude acetonitrile concentration zone 342. Crude acetonitrile concentration zone 342 includes an upper outlet configured to allow sidestream 306 to flow out of column 310 of crude acetonitrile concentration zone 342. Vapor may flow up either side of baffle 344 as stream 304 or as vapor stream 5v. A portion of bottoms stream 302 that is not recycled to the top of column 310 is discharged as stream 307.

The recovery column may include a top section, middle section and bottom section. In one aspect, the top, middle and bottom sections are each about 25 to about 40% of a height (tangent to tangent) of the recovery column. Each recovery column section may be further divided into portions. For example, the middle section of the recovery column includes a top portion, middle portion and bottom portion. In this aspect, the top portion, middle portion and bottom portion are each about 25 to about 40% of a height of the middle section of the recovery column. In another aspect, the top section of the recovery column includes a top portion and a bottom portion that are each about 40 to about 60% of a height of the top section of the recovery column. In another aspect, the bottom section of the recovery column includes a top portion and a bottom portion that are each about 40 to about 60% of a height of the bottom section of the recovery column.

In another aspect, the recovery column may include about 80 to about 120 trays, and in another aspect, about 80 to about 100 trays. The plurality of trays in the recovery column includes a top section of trays, a middle section of trays, and a bottom section of trays. In this aspect, the top section of trays, middle section of trays and bottom section or trays are each about 25 to about 40% of a total number of trays in the recovery column.

The top section of trays of the recovery column includes a top portion of trays and a bottom portion of trays that are each about 40 to about 60% of a total number of trays in the top section of trays in the recovery column. The middle section of trays includes a top portion of trays, middle portion of trays and bottom portion of trays. The top portion of trays, middle portion of trays and bottom portion of trays are each about 25 to about 40% of a total number of trays of the middle section of trays of the recovery column. The bottom section of trays of the recovery column includes a top portion of trays and a bottom portion of trays that are each about 40 to about 60% of a total number of trays in the bottom section of trays in the recovery column.

### Recovery Column Operation

A feed stream provided to the recovery column typically contains acrylonitrile, hydrogen cyanide (HCN), acetonitrile, and water (shown as feed stream 1 in FIG. 1, as feed stream 101 in FIG. 2, and as feed stream 301 in FIG. 3). In this aspect, the feed stream provided to the recovery column includes about 2 to about 10 weight % acrylonitrile, in another aspect, about 3 to about 7 weight %, and in another aspect, about 4 to about 6 weight % acrylonitrile. The mixture also includes acetonitrile in an amount of about 0.1 to about 0.3 weight % acetonitrile, and in another aspect, about 0.15 to about 0.25 weight % acetonitrile. The mixture may also include other components, such as for example, acrolein and/or oxazole in smaller amounts. The feed stream provided to the recovery column has a temperature of about 162 °F (72.2 °C) to about 175 °F (79.4 °C), in another aspect, about 165 °F (73.8 °C) to about 167 °F (75 °C), in another aspect, about 165 °F (73.8 °C) to about 166 °F (74.4 °C), and in another aspect, about 166 °F (74.4 °C) to about 167 °F (75 °C).

Aqueous solvent, which may be solvent water, enters the recovery column at a top portion of the column (shown as water stream 2 in FIG. 1, water stream 102 in FIG. 2 and water stream 302 in FIG. 3). Solvent water which is introduced to the top of the recovery column flows down the tower across the trays, condensing and extracting acetonitrile as it travels to the bottom of the recovery column. As the liquid descends across the trays in the recovery column to the tower bottom, hot vapor from a stripper and heat from the live steam added, travel up the tower through the tray holes allowing intimate contact with the liquid on them. Heat transfer takes place between the liquid and vapor traffic and tends to move all the organics (except acetonitrile) up to the column top. The temperature of aqueous solvent entering the recovery column may be about 102 °F (38.8 °C) to about 128 °F (53.3 °C), in another aspect, about 102 °F (38.8 °C) to about 108 °F (42.2 °C), in another aspect, about 116 °F (46.6 °C) to about 128 °F (53.3 °C), and in another aspect, about 105 °F (40.5 °C) to about 106 °F (41.1 °C).

The overhead stream produced by the recovery column (shown as stream 3 in FIG. 1, stream 103 in FIG. 2, and stream 303 in FIG. 3) may include acrylonitrile, HCN, and a portion of the water from the feed stream. In one aspect, oxazole in the overhead stream is maintained at a concentration of about 30 ppm or less, in another aspect, about 25 ppm or less, in another aspect, about 15 ppm or less, in another aspect, about 30 to about 0.5 ppm, in another aspect, about 30 to about 0.5 ppm, in another aspect, about 25 to about 0.5 ppm, in another aspect, about 25 to about 5 ppm, in another aspect, about 15 to about 0.5 ppm, and in another aspect, about 15 to about 5 ppm.

The recovery column may provide a vapor sidestream that includes water and acetonitrile that is substantially free of acrylonitrile and HCN (shown as stream 104 in FIG. 2, and 304 in FIG. 3). In one aspect, a ratio of the sidestream flow in MSCFH (MSCFH - million standard cubic feet per hour (28320 m³/hr)) to column feed (mixture that includes acrylonitrile and acetonitrile) in U.S gallons per minute (0.0038 m³/hr) is about 0.1 to about 0.3, in another aspect, about 0.1 to about 0.2125, in another aspect, about 0.1 to about 0.2, in another aspect, about 0.2 to about 0.225, and in another aspect, about 0.2 to about 0.2125. In another aspect, HCN in the side stream is maintained at a concentration of about 2 weight percent or less, in another aspect, about 1 weight percent or less, in another aspect, about 0.5 weight percent or less, in another aspect, about 2 to about 0.1 weight percent, and in another aspect, about 1 to about 0.1 weight percent.

The recovery column may also provide a bottoms stream (shown as bottoms stream 2 in FIG. 1, bottoms stream 102 in FIG. 2, and bottoms stream 302 in FIG. 3) which exits from a bottom portion of the recovery column. At least a portion of the bottoms stream may be recycled back to a top portion of the recovery column. Known methods for heating the recovery column include for example, steam, steam heated reboiler and/or heat exchange with other process streams.

In one aspect, the process includes controlling flow rates to provide a ratio of the aqueous solvent flow rate to a flow rate of the mixture that includes acrylonitrile and acetonitrile is about 1:2 to about 1:1.6. The process may further include controlling flow rates to provide a ratio of the sidestream flow rate to a flow rate of the mixture that includes acrylonitrile and acetonitrile is about 1:1.25 to about 1:1.7. In another aspect, the column has a pressure drop of about 16 psid (pounds per square inch differential (110.32 kPa)) or less, and in another aspect, about 12 psid (82.7 kPa) or less. In this aspect, psid represents the pressure difference between the top and bottom of the column.

### Temperature Control

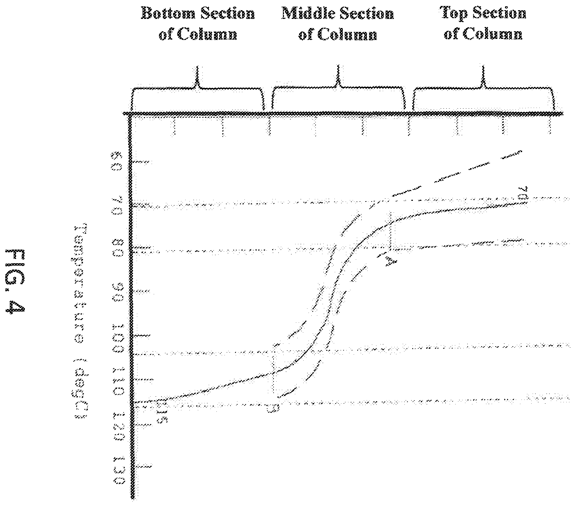

Figure 4 describes a temperature profile for recovery column operation. Proper temperature control and a temperature profile as described in Figure 4 are important for proper column operation. Variations within the temperature profile as defined by the dotted lines in Figure 4 can be effected by a combination of changes involving solvent water and feed temperatures, solvent water rate, and steam rate. Column pressure can also change the profile.

Changes above point "A" on the curve are predominantly due to variations in composition and pressure. Temperature variations above point "A" are not critical, but the overhead temperature should be kept as low as possible to minimize amounts of oxazole, acetonitrile, acetone and water going overhead. The temperature variations below point "B" are predominantly due to tray pressure changes, since the composition in this section is mostly water.

Temperature control of the recovery column includes known temperature control systems that can include reboilers and heat exchangers. In one aspect, the heat duty required to produce the necessary boil-up in the bottom of the recovery column may be provided by heat transfer in any conventional reboiling apparatus. Conventional reboilers may include some variant of a shell-and-tube exchanger. Some examples of reboiler configurations include kettle, thermosyphon, forced circulation, stab-in bundle, horizontal, vertical and falling film. In one aspect, the process includes controlling temperature by removing liquid at or near the bottom of the recovery column and exchanging the liquid in a thermosiphon reboiler. In this aspect, the effluent from the thermosiphon reboiler is returned to the recovery column. Live steam may be injected either to supplement or to replace the required heat duty of the recovery column. In another aspect, the process includes reboiling of the recovery column through two vertical thermosyphon reboilers in parallel, using pressurized steam derived from turbine exhaust.

In one aspect, recovery column temperature is maintained as follows:
the top portion of the middle section of the recovery column is maintained at a temperature of about 65 to about 85 °C, and in another aspect, about 70 to about 80 °C;
the bottom portion of the middle section of the recovery column is maintained at about 100 to about 120 °C, and in another aspect, about 105 to about 115 °C;
the top portion of the top section of the recovery column is maintained at about 55 to about 80 °C, and in another aspect, about 60 to about 75 °C;
the top portion and bottom portion of the top section of the recovery column have a temperature difference of about 0 to about 20 °C, and in another aspect, about 5 to about 15 °C;
the bottom portion of the bottom section of the recovery column is maintained at about 105 to about 125 °C, and in another aspect, about 110 to about 120 °C; and
the top portion and bottom portion of the bottom section of the recovery column have a temperature difference of about 0 to about 15 °C, and in another aspect, about 7 to about 13 °C.

In another aspect, recovery column temperature is maintained as follows:
the top portion of trays of the middle section of the recovery column is maintained at a temperature of about 65 to about 85 °C, and in another aspect, about 70 to about 80 °C;
the bottom portion of trays of the middle section of the recovery column is maintained at about 100 to about 120 °C, and in another aspect, about 105 to about 115 °C;
the top portion of trays of the top section of the recovery column is maintained at about 55 to about 80 °C, and in another aspect, about 60 to about 75 °C;
the top portion of trays and bottom portion of trays of the top section of the recovery column have a temperature difference of about 0 to about 20 °C, and in another aspect, about 5 to about 15 °C;
the bottom portion of trays of the bottom section of the recovery column is maintained at about 105 to about 125 °C, and in another aspect, about 110 to about 120 °C; and
the top portion of trays and bottom portion of trays of the bottom section of the recovery column have a temperature difference of about 0 to about 15 °C, and in another aspect, about 7 to about 13 °C.

In another aspect, temperature is controlled in the middle section of the recovery column as follows:
in one aspect, a temperature drop in the middle section of the recovery column is about 35% or greater of a temperature drop from the top tray to the bottom tray of the recovery column;
in another aspect, the temperature drop in the middle section of the recovery column is about 50% or greater of the temperature drop from the top tray to the bottom tray of the recovery column;
in another aspect, the temperature drop in the middle section of the recovery column is about 75% or greater of the temperature drop from the top tray to the bottom tray of the recovery column;
in another aspect, the temperature drop in the middle section of the recovery column is about 75% of the temperature drop from the top tray to the bottom tray of the recovery column; and
in another aspect, the temperature drop in the middle section of the recovery column is about 80% of the temperature drop from the top tray to the bottom tray of the recovery column.

Temperature control of the recovery column provides an overhead stream, bottoms stream and sidestream with the following compositions:
an overhead stream that includes the acrylonitrile-water azeotrope and 0.05 weight percent or less acetonitrile, in another aspect, about 0.03 weight percent or less acetonitrile, and in another aspect, about 0.01 weight percent or less acetonitrile;
an overhead stream that includes 70 weight percent to 90 weight percent acrylonitrile, and in another aspect, about 75 to about 85 weight percent acrylonitrile;
a bottoms stream that includes 0 to 0.0075 weight percent acetonitrile, in another aspect, about 0.0025 to about 0.007 weight percent acetonitrile, and in another aspect, about 0.0025 to about 0.005 weight percent acetonitrile; and
a sidestream that includes 5 to 70 weight percent acetonitrile, in another aspect, about 5 to about 50 weight percent acetonitrile, and in another aspect, about 6 to about 12 weight percent acetonitrile.

### Advanced Process Control

One objective of a process for extractive distillation of a mixture of acrylonitrile and acetonitrile includes reducing impurities in an overhead stream and in a sidedraw. At a first control aspect, control of impurity levels includes an amount of water added to the recovery column and a column temperature. In another control aspect, control includes a level or amount of material in a decanter and a pressure drop across the recovery column. Further controls may include a level of oxazole impurity in the overhead stream, HCN levels in the sidedraw, a solvent water to rich water flow ratio, and a sidedraw to rich water flow ratio.

In one aspect, control of the process includes using an advanced process controller based on model predictive control to determine simultaneous control actions for a set of manipulated variables in order to optimize at least one of a set of parameters whilst controlling at least one set of controlled variable. In this aspect, the set of manipulated variable includes an amount of aqueous solvent added to the distillation column, and the distillation column temperature. The set of control variables includes an amount of acetonitrile in the overhead and an amount of acrylonitrile in the sidestream. The set of control variable may further include a decanter level and/or a distillation column pressure drop. In another aspect, the controlled variables further includes parameters that include a level of oxazole in the overhead, a ratio of an aqueous solvent flow rate to a flow rate of the mixture that includes acrylonitrile and acetonitrile, a ratio of the sidestream flow rate to a flow rate of the mixture that includes acrylonitrile and acetonitrile, and mixtures thereof.

As used herein, the term "manipulated variable" refers to variables that are adjusted by the advanced process controller. The term "controlled variables" refers to variable that are kept by the advanced process controller at a predetermined value (set point) or within a predetermined range (set range). "Optimizing a variable" refers to maximizing or minimizing the variable and to maintaining the variable at a predetermined value.

One aspect of model predictive control is that future process behavior is predicted using a model and available measurements of the controlled variables. The controller outputs are calculated so as to optimize a performance index, which is a linear or quadratic function of the predicted errors and calculated future control moves. At each sampling instant, the control calculations are repeated and predictions updated based on current measurements. In this aspect, a suitable model is one that includes a set of empirical step-response models expressing the effects of a step-response of a manipulated variable on the controlled variables.

An optimum value for the parameter to be optimized can be obtained from a separate optimization step, or the variable to be optimized can be included in the performance function.

Before model predictive control can be applied, one determines first the effect of step changes of the manipulated variables on the variable to be optimized and on the controlled variables. This results in a set of step-response coefficients. This set of step-response coefficients forms the basis of the model predictive control of the process.

During normal operation, the predicted values of the controlled variables are regularly calculated for a number of future control moves. For these future control moves a performance index is calculated. The performance index includes two terms, a first term representing the sum over the future control moves of the predicted error for each control move and a second term representing the sum over the future control moves of the change in the manipulated variables for each control move. For each controlled variable, the predicted error is the difference between the predicted value of the controlled variable and a reference value of the controlled variable. The predicted errors are multiplied with a weighting factor, and the changes in the manipulated variables for a control move are multiplied with a move suppression factor. The performance index discussed here is linear.

Alternatively, the terms may be a sum of squared terms, in which case the performance index is quadratic. Moreover, constraints can be set on manipulated variables, change in manipulated variables and on controlled variables. This results in a separate set of equations that are solved simultaneously with the minimization of the performance index.

Optimization can be done in two ways; one way is to optimize separately, outside the minimization of the performance index, and the second way is to optimize within the performance index.

When optimization is done separately, the variables to be optimized are included as controlled variables in the predicted error for each control move and the optimization gives a reference value for the controlled variables.

Alternatively, optimization is done within the calculation of the performance index, and this gives a third term in the performance index with an appropriate weighting factor. In this case, the reference values of the controlled variables are pre-determined steady state values, which remain constant.

The performance index is minimized taking into account the constraints to give the values of the manipulated variables for the future control moves. However, only the next control move is executed. Then the calculation of the performance index for future control moves starts again.

The models with the step response coefficients and the equations required in model predictive control are part of a computer program that is executed in order to control the liquefaction process. A computer program loaded with such a program that can handle model predictive control is called an advanced process controller. Commercially available computer programs that may be utilized include for example, DMCplus^{®} by Aspen Technology and PredictPro^{®} by Emerson.

In one aspect, the process provides an increase in column capacity. Column capacity is defined as an amount of rich water (mixture that includes acrylonitrile and acetonitrile provided to the column) the column can process. In this aspect, the process provides a ratio of column capacity (tons/hour) to cross sectional area (at a level of the feed inlet in mm²) of about 0.00002 to about 0.00003.

In another aspect, the process provides a decrease in energy usage. In this aspect, the process provides an energy usage of 1600 btu or less per gallon (1688089 Joule or less per 3785 ml) of the mixture that includes acrylonitrile and acetonitrile provided to the column, in another aspect, about 1500 btu or less, (about 1582584 Joule or less) and in another aspect, about 1400 btu or less per gallon (about 1477078 Joule or less per 3785ml) of the mixture that includes acrylonitrile and acetonitrile provided to the column.

## Claims

1. An extractive distillation process comprising:
providing a mixture that includes acrylonitrile and acetonitrile to at least one distillation column;
contacting the mixture that includes acrylonitrile and acetonitrile with aqueous solvent to provide an acrylonitrile-water azeotrope; and
separating the acrylonitrile-water azeotrope from the acetonitrile to provide an overhead stream, a sidestream and a bottoms stream, wherein the overhead stream comprises the acrylonitrile-water azeotrope, 0.05 weight percent or less acetonitrile, and 70 to 90 weight percent acrylonitrile, the bottoms stream comprises 0 to 0.0075 weight percent acetonitrile, and the side stream comprises 5 to 70 weight percent acetonitrile and 1 weight percent or less acrylonitrile,
wherein the weight percent acetonitrile in the overhead stream and the weight percent acrylonitrile in the sidestream are controlled based on the flow of the aqueous solvent added to the distillation column and a temperature of the distillation column.

2. An extractive distillation column control process comprising:
providing a mixture that includes acrylonitrile and acetonitrile to at least one distillation column;
contacting the mixture that includes acrylonitrile and acetonitrile with aqueous solvent to provide an acrylonitrile-water azeotrope;
separating the acrylonitrile-water azeotrope from the acetonitrile to provide an overhead stream, a sidestream and a bottoms stream, wherein the overhead stream comprises the acrylonitrile-water azeotrope, 0.05 weight percent or less acetonitrile, and 70 to 90 weight percent acrylonitrile, the bottoms stream comprises 0 to 0.0075 weight percent acetonitrile, and the side stream comprises 5 to 70 weight percent acetonitrile and 1 weight percent or less acrylonitrile; and
controlling an amount of acetonitrile in the overhead stream and the amount of acrylonitrile in the sidestream using an advanced process controller based on model predictive control to determine simultaneous control actions for manipulated variables in order to optimize at least one set of parameters while controlling at least one set of controlled variables,
wherein the set of manipulated variables includes an amount of the aqueous solvent added to the distillation column and distillation column temperature and the set of controlled variable includes the amount of acetonitrile in the overhead and the amount of acrylonitrile in the sidestream,
wherein the controlling of the at least one set of controlled variables includes controlling the amount of aqueous solvent added to the distillation column and the distillation column temperature.

3. The process of claim 1 wherein the temperature of the distillation column is measured at a middle section of the column.

4. The process of claim 1 or claim 2 wherein the overhead stream is sent to a decanter and a volume of the decanter is maintained at 50 percent to 70 percent of a daily average volume of the decanter.

5. The process of claim 1 or claim 2 wherein an organic flow from the decanter is 6 to 11 percent of a volume of the mixture that includes acrylonitrile and acetonitrile.

6. The process of claim 1 or claim 2 wherein the distillation column has a pressure drop of 110.32 kPa differential (16 psid) or less.

7. The process of claim 1 or claim 2 wherein a ratio of the sidestream flow rate in 28320 m³ per hour (million standard cubic feet per hour (MSCFH)) to a feed rate of the mixture that includes acrylonitrile and acetonitrile in 0.0038 m³ per minute (gallons per minute) is 0.1 to 0.3.

8. The process of claim 1 or claim 2 wherein oxazole in the overhead stream is maintained at a concentration of 30 ppm or less.

9. The process of claim 1 or claim 2 wherein HCN in the sidestream is maintained at a concentration of 2 weight percent or less.

10. The process of claim 1 or claim 2 wherein a ratio of the aqueous solvent flow rate to a flow rate of the mixture that includes acrylonitrile and acetonitrile is 1:2 to 1:1.6.

11. The process of claim 1 or claim 2 wherein a ratio of the sidestream flow rate to a flow rate of the mixture that includes acrylonitrile and acetonitrile is 1:1.25 to 1:1.7.

12. The process of claim 1 or claim 2 wherein the process provides an energy usage of 1600 btu or less per gallon (1688089 Joule or less per 3785 ml) of the mixture of acrylonitrile and acetonitrile provided to the recovery column.

13. The process of claim 2 wherein the set of controlled variable further includes a decanter level and/or a distillation column pressure drop.

14. The process of claim 13 wherein the set of controlled variables further includes parameters selected from the group consisting of a level of oxazole in the overhead, a ratio of an aqueous solvent flow rate to a flow rate of the mixture that includes acrylonitrile and acetonitrile, a ratio of the sidestream flow rate to a flow rate of the mixture that includes acrylonitrile and acetonitrile, and mixtures thereof.

15. The process of claim 1 or claim 2 wherein the mixture that includes acrylonitrile and acetonitrile has a temperature of 162 °F (72.2 °C) to 175 °F (79.4 °C) prior to entering the distillation column.

16. The process of claim 1 or claim 2 wherein the aqueous solvent has a temperature of 102 °F (38.8 °C) to 128 °F (53.3 °C) prior to entering the distillation column.

## Patentansprüche

1. Extraktivdestillationsverfahren, umfassend:
Bereitstellen einer Mischung, die Acrylnitril und Acetonitril enthält, an mindestens eine Destillationskolonne;
Inkontaktbringen der Mischung, die Acrylnitril und Acetonitril enthält, mit wässrigem Lösungsmittel, um ein Acrylnitril-Wasser-Azeotrop bereitzustellen; und
Trennen des Acrylnitril-Wasser-Azeotrops von dem Acetonitril, um einen Kopfstrom, einen Seitenstrom und einen Bodenstrom bereitzustellen, wobei der Kopfstrom das Acrylnitril-Wasser-Azeotrop, 0,05 Gewichtsprozent oder weniger Acetonitril und 70 bis 90 Gewichtsprozent Acrylnitril umfasst, der Bodenstrom 0 bis 0,0075 Gewichtsprozent Acetonitril umfasst und der Seitenstrom 5 bis 70 Gewichtsprozent Acetonitril und 1 Gewichtsprozent oder weniger Acrylnitril umfasst,
wobei der Gewichtsprozentsatz an Acetonitril in dem Kopfstrom und der Gewichtsprozentsatz an Acrylnitril in dem Seitenstrom basierend auf dem Fluss des wässrigen Lösungsmittels, das der Destillationskolonne zugesetzt wird, und einer Temperatur der Destillationskolonne gesteuert werden.

2. Verfahren zur Steuerung einer Extraktivdestillationskolonne, umfassend:
Bereitstellen einer Mischung, die Acrylnitril und Acetonitril enthält, an mindestens eine Destillationskolonne;
Inkontaktbringen der Mischung, die Acrylnitril und Acetonitril enthält, mit wässrigem Lösungsmittel, um ein Acrylnitril-Wasser-Azeotrop bereitzustellen;
Trennen des Acrylnitril-Wasser-Azeotrops von dem Acetonitril, um einen Kopfstrom, einen Seitenstrom und einen Bodenstrom bereitzustellen, wobei der Kopfstrom das Acrylnitril-Wasser-Azeotrop, 0,05 Gewichtsprozent oder weniger Acetonitril und 70 bis 90 Gewichtsprozent Acrylnitril umfasst, der Bodenstrom 0 bis 0,0075 Gewichtsprozent Acetonitril umfasst und der Seitenstrom 5 bis 70 Gewichtsprozent Acetonitril und 1 Gewichtsprozent oder weniger Acrylnitril umfasst; und
Steuern einer Menge an Acetonitril im Kopfstrom und der Menge an Acrylnitril im Seitenstrom unter Verwendung einer fortschrittlichen Verfahrenssteuerung, die auf modellprädiktiver Steuerung basiert, um gleichzeitige Steuerungsmaßnahmen für Stellgrößen zu bestimmen, um mindestens einen Satz Parameter zu optimieren und gleichzeitig mindestens einen Satz Regelgrößen zu steuern,
wobei der Satz Stellgrößen eine Menge des der Destillationskolonne zugesetzten wässrigen Lösungsmittels und die Temperatur der Destillationskolonne beinhaltet und der Satz Regelgrößen die Menge an Acetonitril in dem Kopfprodukt und die Menge an Acrylnitril in dem Seitenstrom beinhaltet,
wobei das Steuern des mindestens einen Satzes Regelgrößen Steuern der Menge an der Destillationskolonne zugesetztem wässrigem Lösungsmittel und die Temperatur der Destillationskolonne beinhaltet.

3. Verfahren nach Anspruch 1, wobei die Temperatur der Destillationskolonne in einem mittleren Abschnitt der Kolonne gemessen wird.

4. Verfahren nach Anspruch 1 oder Anspruch 2, wobei der Kopfstrom zu einem Dekanter geleitet wird und ein Volumen des Dekanters bei 50 Prozent bis 70 Prozent des durchschnittlichen Tagesvolumens des Dekanters gehalten wird.

5. Verfahren nach Anspruch 1 oder Anspruch 2, wobei ein Fluss organischer Stoffe aus dem Dekanter 6 bis 11 Prozent des Volumens der Mischung beträgt, die Acrylnitril und Acetonitril enthält.

6. Verfahren nach Anspruch 1 oder Anspruch 2, wobei die Destillationskolonne einen Druckabfall von 110,32 kPa Differenzdruck (16 psid) oder weniger aufweist.

7. Verfahren nach Anspruch 1 oder Anspruch 2, wobei ein Verhältnis der Seitenstromflussrate in 28320 m³ pro Stunde (Millionen Standardkubikfuß pro Stunde (MSCFH)) zu einer Zufuhrrate der Mischung, die Acrylnitril und Acetonitril enthält, in 0,0038 m³ pro Minute (Gallonen pro Minute) 0,1 bis 0,3 beträgt.

8. Verfahren nach Anspruch 1 oder Anspruch 2, wobei die Oxazolkonzentration im Kopfstrom bei einer Konzentration von 30 ppm oder weniger gehalten wird.

9. Verfahren nach Anspruch 1 oder Anspruch 2, wobei die HCN-Konzentration in dem Seitenstrom bei einer Konzentration von 2 Gewichtsprozent oder weniger gehalten wird.

10. Verfahren nach Anspruch 1 oder Anspruch 2, wobei ein Verhältnis der Flussrate des wässrigen Lösungsmittels zu einer Flussrate der Mischung, die Acrylnitril und Acetonitril enthält, 1:2 bis 1:1,6 beträgt.

11. Verfahren nach Anspruch 1 oder Anspruch 2, wobei ein Verhältnis der Seitenstrom-Flussrate zu einer Flussrate der Mischung, die Acrylnitril und Acetonitril enthält, 1:1,25 bis 1:1,7 beträgt.

12. Verfahren nach Anspruch 1 oder Anspruch 2, wobei das Verfahren einen Energieverbrauch von 1600 BTU oder weniger pro Gallone (1688089 Joule oder weniger pro 3785 ml) der der Rückgewinnungskolonne bereitgestellten Mischung aus Acrylnitril und Acetonitril bereitstellt.

13. Verfahren nach Anspruch 2, wobei der Satz gesteuerter Variablen außerdem einen Dekanterfüllstand und/oder einen Druckabfall in der Destillationskolonne umfasst.

14. Verfahren nach Anspruch 13, wobei der Satz Regelgrößen ferner Parameter beinhaltet, die aus der Gruppe ausgewählt sind, die aus einem Oxazolgehalt in dem Kopfprodukt, einem Verhältnis einer Flussrate des wässrigen Lösungsmittels zu einer Flussrate der Mischung, die Acrylnitril und Acetonitril enthält, einem Verhältnis der Seitenstromflussrate zu einer Flussrate der Mischung, die Acrylnitril und Acetonitril enthält, sowie Mischungen davon besteht.

15. Verfahren nach Anspruch 1 oder Anspruch 2, wobei die Mischung, die Acrylnitril und Acetonitril enthält, vor dem Eintritt in die Destillationskolonne eine Temperatur von 162 °F (72,2 °C) bis 175 °F (79,4 °C) aufweist.

16. Verfahren nach Anspruch 1 oder Anspruch 2, wobei das wässrige Lösungsmittel vor dem Eintritt in die Destillationskolonne eine Temperatur von 102 °F (38,8 °C) bis 128 °F (53,3 °C) aufweist.

## Revendications

1. Procédé de distillation extractive comprenant :
la fourniture d'un mélange qui comprend de l'acrylonitrile et de l'acétonitrile à au moins une colonne de distillation ;
la mise en contact du mélange qui comprend de l'acrylonitrile et de l'acétonitrile avec un solvant aqueux pour fournir un azéotrope acrylonitrile-eau ; et
la séparation de l'azéotrope acrylonitrile-eau de l'acétonitrile pour fournir un courant de tête, un courant latéral et un courant de queue, dans lequel le courant de tête comprenant l'azéotrope acrylonitrile-eau, 0,05 pour cent en poids ou moins d'acétonitrile et 70 à 90 pour cent en poids d'acrylonitrile, le courant de queue comprend 0 à 0,0075 pour cent en poids d'acétonitrile, et le courant latéral comprend 5 à 70 pour cent en poids d'acétonitrile et 1 pour cent en poids ou moins d'acrylonitrile,
dans lequel le pourcentage en poids d'acétonitrile dans le courant de tête et le pourcentage en poids d'acrylonitrile dans le courant latéral sont régulés sur la base du flux du solvant aqueux ajouté à la colonne de distillation et d'une température de la colonne de distillation.

2. Procédé de régulation d'une colonne de distillation extractive comprenant :
la fourniture d'un mélange qui comprend de l'acrylonitrile et de l'acétonitrile à au moins une colonne de distillation ;
la mise en contact du mélange qui comprend de l'acrylonitrile et de l'acétonitrile avec un solvant aqueux pour fournir un azéotrope acrylonitrile-eau ;
la séparation de l'azéotrope acrylonitrile-eau de l'acétonitrile pour fournir un courant de tête, un courant latéral et un courant de queue, dans lequel le courant de tête comprenant l'azéotrope acrylonitrile-eau, 0,05 pour cent en poids ou moins d'acétonitrile et 70 à 90 pour cent en poids d'acrylonitrile, le courant de queue comprend 0 à 0,0075 pour cent en poids d'acétonitrile, et le courant latéral comprend 5 à 70 pour cent en poids d'acétonitrile et 1 pour cent en poids ou moins d'acrylonitrile ; et
la régulation d'une quantité d'acétonitrile dans le courant de tête et de la quantité d'acrylonitrile dans le courant latéral à l'aide d'un régulateur de processus avancé basé sur la régulation prédictive de modèle pour déterminer des actions de régulation simultanées pour les variables manipulées afin d'optimiser au moins un ensemble de paramètres tout en régulant au moins un ensemble de variables régulées,
dans lequel l'ensemble de variables manipulées comprend une quantité du solvant aqueux ajouté à la colonne de distillation et la température de la colonne de distillation et l'ensemble de variables régulées comprend la quantité d'acétonitrile dans le courant de tête et la quantité d'acrylonitrile dans le courant latéral,
dans lequel la régulation de l'au moins un ensemble de variables régulées comprend la régulation de la quantité de solvant aqueux ajoutée à la colonne de distillation et de la température de la colonne de distillation.

3. Procédé selon la revendication 1, dans lequel la température de la colonne de distillation est mesurée au niveau d'une section médiane de la colonne.

4. Procédé selon la revendication 1 ou la revendication 2, dans lequel le courant de tête est envoyé à un décanteur et un volume du décanteur est maintenu à 50 pour cent à 70 pour cent d'un volume moyen quotidien du décanteur.

5. Procédé selon la revendication 1 ou la revendication 2, dans lequel un flux organique provenant du décanteur est de 6 à 11 pour cent d'un volume du mélange qui comprend de l'acrylonitrile et de l'acétonitrile.

6. Procédé selon la revendication 1 ou la revendication 2, dans lequel la colonne de distillation a une perte de charge de 110,32 kPa différentiel (16 psid) ou moins.

7. Procédé selon la revendication 1 ou la revendication 2, dans lequel un rapport du débit du courant latéral dans 28 320 m³ par heure (million de pieds cubes standard par heure (MSCFH)) à un débit d'alimentation du mélange qui comprend de l'acrylonitrile et de l'acétonitrile dans 0,0038 m³ par minute (gallons par minute) est de 0,1 à 0,3.

8. Procédé selon la revendication 1 ou la revendication 2, dans lequel l'oxazole dans le courant de tête est maintenu à une concentration de 30 ppm ou moins.

9. Procédé selon la revendication 1 ou la revendication 2, dans lequel le HCN dans le courant latéral est maintenu à une concentration de 2 pour cent en poids ou moins.

10. Procédé selon la revendication 1 ou la revendication 2, dans lequel un rapport du débit du solvant aqueux à un débit du mélange qui comprend de l'acrylonitrile et de l'acétonitrile est de 1:2 à 1:1,6.

11. Procédé selon la revendication 1 ou la revendication 2, dans lequel un rapport du débit du courant latéral à un débit du mélange qui comprend de l'acrylonitrile et de l'acétonitrile est de 1:1,25 à 1:1,7.

12. Procédé selon la revendication 1 ou la revendication 2, dans lequel le procédé fournit une consommation d'énergie de 1 600 btu ou moins par gallon (1 688 089 Joules ou moins par 3 785 ml) du mélange d'acrylonitrile et d'acétonitrile fourni à la colonne de récupération.

13. Procédé selon la revendication 2, dans lequel l'ensemble de variables régulées comprend en outre un niveau de décantation et/ou une perte de charge à travers la colonne de distillation.

14. Procédé selon la revendication 13, dans lequel l'ensemble de variables régulées comprend en outre des paramètres choisis dans le groupe constitué par un niveau d'oxazole dans la tête, un rapport d'un débit du solvant aqueux à un débit du mélange qui comprend de l'acrylonitrile et de l'acétonitrile, un rapport du débit de courant latéral à un débit du mélange qui comprend de l'acrylonitrile et de l'acétonitrile, et des mélanges de ceux-ci.

15. Procédé selon la revendication 1 ou la revendication 2, dans lequel le mélange qui comprend de l'acrylonitrile et de l'acétonitrile a une température de 162 °F (72,2 °C) à 175 °F (79,4 °C) avant d'entrer dans la colonne de distillation.

16. Procédé selon la revendication 1 ou la revendication 2, dans lequel le solvant aqueux a une température de 102 °F (38,8°C) à 128 °F (53,3 °C) avant d'entrer dans la colonne de distillation.
